# FASCICULE DE BREVET EUROPEEN

(11) **EP 4 215 799 B1**
(45) Date de publication et mention de la délivrance du brevet: **06.08.2025**
(21) Numéro de dépôt: 22211128.8
(22) Date de dépôt: 02.12.2022
(51) Int. Cl.: F17C 13/04

(54) **CARTOUCHE DE STOCKAGE D'UN MÉLANGE NO/AZOTE ET INSTALLATION DE FOURNITURE DE GAZ ASSOCIÉE**
KARTUSCHE ZUR LAGERUNG EINES NO/STICKSTOFF-GEMISCHES UND ZUGEHÖRIGE GASBEREITSTELLUNGSANLAGE
CARTRIDGE FOR STORING A NO/NITROGEN MIXTURE AND ASSOCIATED GAS SUPPLY INSTALLATION

(30) Priorité: 24.01.2022 FR 2200573
(43) Date de publication de la demande: 26.07.2023
(73) Titulaire: Inosystems, 92160 Antony (FR)
(72) Inventeur: BOULANGER, Thierry, 92160 Antony (FR); MARCHAL, Frédéric, 92160 Antony (FR)
(74) Mandataire: Air Liquide

(56) Documents cités:
- EP-B1- 3 466 473
- FR-A1- 2 975 010
- FR-A1- 3 042 584
- GB-A- 2 096 299
- KR-A- 20180 072 958
- US-A1- 2017 095 634
- US-A1- 2018 022 537

## Description

L'invention concerne une cartouche de stockage, i.e. de conditionnement, et son utilisation pour le stockage d'un mélange gazeux de monoxyde d'azote et d'azote (NO/N₂), laquelle cartouche est conçue pour être connectée fluidiquement à un dispositif de délivrance de NO gazeux servant à injecter le mélange gazeux NO/N₂ dans le circuit ventilatoire d'un ventilateur médical, c'est-à-dire un appareil médical d'administration de gaz à un patient, ainsi que l'utilisation d'une telle cartouche pour conditionner un mélange gazeux NO/N₂, ainsi qu'une installation de fourniture de gaz à un patient comprenant une telle cartouche alimentant un dispositif de délivrance de NO et un ventilateur médical alimenté en mélange gazeux NO/N₂ par ledit dispositif de délivrance de NO.

Le monoxyde d'azote ou NO est un gaz qui, lorsqu'inhalé, dilate les vaisseaux pulmonaires et augmente l'oxygénation en améliorant les échanges gazeux. Les propriétés du NO sont utilisées pour traiter différentes conditions médicales, comme l'Hypertension Artérielle Pulmonaire du nouveau-né ou PPHN (pour *Persistent Pulmonary Hypertension of the Newborn*), le Syndrome de Détresse Respiratoire Aigüe ou SDRA observé principalement chez l'adulte ou encore les hypertensions pulmonaires en chirurgie cardiaque ou autres, comme enseigné notamment par EP-A-560928, EP-A-1516639 ou US-A-10,201,564.

En général, le mélange NO/N₂ contenant une faible quantité de NO gazeux (i.e. quelques dizaines ou centaines de ppm en vol.) est injecté, via un dispositif de délivrance de NO gazeux, dans un flux gazeux contenant de l'oxygène (O₂) circulant dans le circuit ventilatoire d'un ventilateur médical et qui est ensuite inhalé par le patient. Typiquement, le gaz contenant de l'oxygène est typiquement un mélange N₂/O₂ ou de l'air, tel de l'air de qualité médical. Par ailleurs, la concentration de NO inhalée par le patient, qui correspond à une posologie, est déterminée par le médecin ou analogue. En général, la concentration de NO dans le gaz inhalé par le patient est comprise entre 1 et 80 ppm en volume (ppmv), en fonction de la population traitée, i.e. nouveau-nés ou adultes, et donc de la maladie à traiter.

Ainsi, US-A-5,558,083 décrit un dispositif de délivrance de NO associé à un ventilateur mécanique alimentant une interface respiratoire délivrant le NO au patient, par exemple un masque respiratoire, une sonde d'intubation trachéale ou similaire.

Le dispositif de délivrance de NO est fluidiquement connecté et alimenté par une ou plusieurs bouteilles de gaz contenant le mélange de N₂/NO dont la concentration en NO est souvent comprise entre 200 et 1000 ppmv.

De telles bouteilles de gaz se présentent sous la forme de cylindres ou ogives métalliques, typiquement en acier ou alliage d'aluminium, de plusieurs dizaines de centimètres de haut, en général de l'ordre de 1 m de hauteur, renfermant le mélange NO/N₂ à haute pression, c'est-à-dire à plusieurs dizaines, voire centaines de bar, par exemple de l'ordre de 140 à 230 bar.

Par ailleurs, afin de fournir une pression compatible avec le dispositif de délivrance de NO, ces bouteilles sont toujours équipées d'un régulateur de pression permettant d'abaisser la pression présente dans la bouteille en une pression plus faible, dite de service, en sortie du régulateur. Le régulateur de pression est lui-même une pièce métallique, par exemple en acier inoxydable. Les bouteilles de N₂/NO sont donc volumineuses et/ou encombrantes, ce qui peut poser un problème lors de leur utilisation dans les salles de soins critiques qui peuvent être exiguës et déjà très encombrées par d'autres équipements médicaux. De plus, les volumes de gaz importants contenus dans ces bouteilles de NO/N₂ entrainent aussi des contraintes de stockage, nécessitant d'entreposer ces bouteilles dans des salles dédiées et ventilées, ce qui pose un problème de place et de logistique au sein des bâtiments hospitaliers.

Les dimensions et l'architecture des bouteilles équipées de régulateurs de pression et les volumes de gaz qui y sont stockés aboutissent inévitablement à un poids important, typiquement un poids de l'ordre de 20 kg par bouteille. On comprend que de tels poids engendrent des difficultés logistiques et de maniabilité pour le personnel soignant, en particulier lors de transfert intra-hospitalier. D'ailleurs des blessures ont déjà été rapportées, telles que bouteilles tombées sur les pieds d'utilisateurs lors de procédures de remplacement de bouteilles (i.e. vide contre pleine), mal de dos après soulèvement, portage ou manipulation de bouteilles pleines....

Par ailleurs, tout traitement impliquant du NO est critique et ne peut tolérer une interruption brutale de la thérapie, au risque d'observer chez le patient un effet rebond. Dès lors, tout dispositif de délivrance de NO est généralement raccordé à 2 bouteilles de gaz identiques. Lorsqu'une bouteille devient vide, le dispositif de délivrance de NO bascule automatiquement sur la bouteille pleine, ce qui minimise le risque d'interruption mais aggrave la problématique d'encombrement susmentionné dans les environnements hospitaliers exigus, telles les salles de soins critiques.

Enfin, utiliser des bouteilles de gaz ayant une pression interne très élevée, par exemple 140 bars ou plus, requiert un personnel formé, en particulier sur la manipulation des régulateurs de pression, ce qui complexifie la mise en œuvre d'une thérapie à base de NO hors des salles de soins intensifs, par exemple en salle de soins légers, mais aussi entraine des coûts logistiques plus importants, notamment pour ce qui est de l'acheminement des bouteilles du site de production vers le lieu d'utilisation, typiquement un hôpital.

US-A-10,213,572 propose de remplacer les bouteilles de gaz classiques, qui sont lourdes et encombrantes, par une cassette renfermant une petite flasque en verre hermétique contenant du peroxyde d'azote N₂O₄ liquide. Un percuteur permet de libérer le N₂O₄ en brisant la flasque. En chauffant, le N₂O₄ se dissocie en NO₂ qui, mis en contact avec de l'acide ascorbique solide ou analogue, se transforme en NO qui peut ensuite être délivré sous forme de NO pour traiter un patient. Toutefois, ce type de cassette engendre des problèmes de logistique, de stockage, de manipulation et autres, et des risques du fait de la mise en œuvre de produits chimiques toxiques. De plus, leur coût est élevé.

On connait par ailleurs d'autres documents parlant de conditionnement de gaz à haute pression, à savoir GB-A-2096299 et KR-A-2018/0072958 enseignant des cartouches de stockage, notamment de dioxyde de carbone, FR-A-3042584 portant sur le stockage de gaz médicaux dans des récipients de gaz de grandes dimensions, typiquement jusqu'à 20L compatibles avec des pressions très élevées, i.e. jusqu'à 350 bar, et US-A-2018/022537 concernant un conteneur pour substance aérosol.

US 2017/095634 décrit un dispositif de délivrance de NO.

Au vu de cela, il existe un besoin qui est de pouvoir mettre en œuvre des mélanges NO/azote en milieu hospitalier sans rencontrer tout ou partie des problèmes et inconvénients susmentionnés, en particulier de pouvoir se passer des bouteilles de gaz classiques qui sont notamment lourdes et encombrantes, ou de cassettes dont le fonctionnement est basé sur des produits chimiques dangereux et coûteux.

La solution selon l'invention concerne alors une utilisation d'une cartouche de stockage de gaz sous pression selon la revendication 1.

Dans le cadre de l'invention :
- la pression est exprimée en « bar absolu », abrévié « bar »,
- le volume est donné en équivalent en eau en étant exprimé en millilitre, abrévié « mL »,
- les teneurs en NO sont exprimées en partie par million en volume, abrévié « ppmv ».

Selon le mode de réalisation considéré, la cartouche de stockage de l'invention et/ou son utilisation pour stocker le gaz peut comprendre l'une ou plusieurs des caractéristiques suivantes :
- le corps principal est en alliage d'aluminium.
- le corps principal comprend une paroi ayant une épaisseur comprise entre 0,1 et 0,5 mm.
- le corps principal est allongé selon un axe principal (AA).
- le corps principal a une hauteur comprise entre 15 cm et 30 cm.
- le corps principal a une forme d'ogive.
- le corps principal est fermé à son extrémité supérieure, par un couvercle, de préférence un couvercle serti ou soudé à la partie du corps principal en forme d'ogive.
- la valve de distribution est portée par le couvercle.
- la valve de distribution est agencée au centre du couvercle.
- le corps principal s'étend entre un fond et une extrémité supérieure à laquelle est fixé le couvercle.
- la valve de distribution, le couvercle et le corps principal sont coaxiaux.
- le couvercle est en alliage d'aluminium
- elle pèse de préférence entre 150 et 750 grammes.
- la concentration en NO dans le mélange gazeux NO/N₂ est comprise entre 22 500 et 23 500 ppmv, de préférence au plus 23 000 ppmv.
- On ne peut exclure la présence d'impuretés inévitables résultant du procédé de fabrication du mélange gazeux, notamment de la vapeur d'eau et/ou de l'oxygène, en quantités négligeables, typiquement moins de 3 à 5 ppmv.
- le corps principal comprend un fond, une portion tubulaire intermédiaire et une extrémité supérieure fermée par un couvercle portant la valve de distribution de gaz, de préférence la portion tubulaire intermédiaire est sensiblement en forme d'ogive.
- le couvercle est serti ou soudé à la portion tubulaire intermédiaire.
- le couvercle à une périphérie circulaire.
- le couvercle est conformé pour présenter un bourrelet central, i.e. une protubérance faisant saillie vers l'extérieur, c'est-à-dire au-dessus du couvercle, comprenant un passage central et définissant un compartiment interne.
- le passage central du bourrelet central communique avec le compartiment interne dudit bourrelet central.
- la valve de distribution comprend un canal d'échappement associé à un élément de siège, ledit canal d'échappement étant mobile en translation axiale au sein du passage central du bourrelet central, et l'élément de siège coopérant avec un élément d'étanchéité pour opérer une étanchéité gazeuse et empêcher tout passage de gaz du volume interne de la cartouche au canal d'échappement.
- l'élément d'étanchéité est un joint d'étanchéité, de préférence plat, tel un joint torique plat.
- le canal d'échappement et l'élément de siège sont solidaires l'un de l'autre, typiquement fixés l'un à l'autre.
- l'élément de siège est normalement repoussé en direction de l'élément d'étanchéité par un moyen élastique.
- l'élément de siège est normalement repoussé en direction de l'élément d'étanchéité par un moyen élastique par l'intermédiaire d'un élément diffuseur agencé entre l'élément de siège et l'élément d'étanchéité.
- le moyen élastique vient appuyer sur l'élément diffuseur pour le repousser en direction de l'élément de siège.
- le moyen élastique est un ressort à spirales cylindrique.
- l'élément de siège est mobile en translation dans une pièce-support formant un manchon logeant l'élément de siège et le moyen élastique, et préférentiellement l'élément diffuseur.
- la pièce-support est logée et fixée dans le compartiment interne du bourrelet central.
- le canal d'échappement comprend une extrémité aval libre située à l'extérieur du bourrelet central et une extrémité amont située dans le compartiment interne du bourrelet central et coopérant avec l'élément de siège.

Selon encore un autre aspect, l'invention porte également aussi sur une utilisation d'une cartouche de gaz selon l'invention en tant que source de NO pour alimenter en mélange gazeux NO/N₂, un dispositif de délivrance de NO d'une installation de fourniture de gaz à un patient comprenant :
- ladite au moins une source de NO,
- ledit dispositif de délivrance de NO,
- une branche inspiratoire d'un circuit patient alimentée en mélange gazeux NO/N₂ par le dispositif de délivrance de NO, et
- un ventilateur médical en communication fluidique avec la branche inspiratoire pour alimenter ladite branche inspiratoire en un gaz respiratoire contenant au moins 21% d'oxygène, de préférence de l'air ou un mélange oxygène/azote.

L'invention va maintenant être mieux comprise grâce à la description détaillée suivante, faite à titre illustratif mais non limitatif, en référence aux figures annexées parmi lesquelles :
- Fig. 1 représente un mode de réalisation d'une cartouche de stockage utilisée conditionner un mélange NO/N₂,
- Fig. 2 schématise un mode de réalisation d'une valve de distribution de gaz équipant la cartouche de gaz de Fig. 1, représentée en position fermée,
- Fig. 3 schématise la valve de distribution de gaz de Fig. 2, en position ouverte,
- Fig. 4 schématise un mode de réalisation d'une installation de délivrance de NO à un patient incorporant une cartouche de stockage utilisée pour stocker un mélange NO/N₂, telle celle de Fig. 1
- Fig. 5 schématise un mode de réalisation de l'élément de siège de la valve de distribution de gaz de Fig. 2 et Fig. 3, et
- Fig. 6 schématise un mode de réalisation de l'élément diffuseur de la valve de distribution de gaz de Fig. 2 et Fig. 3.

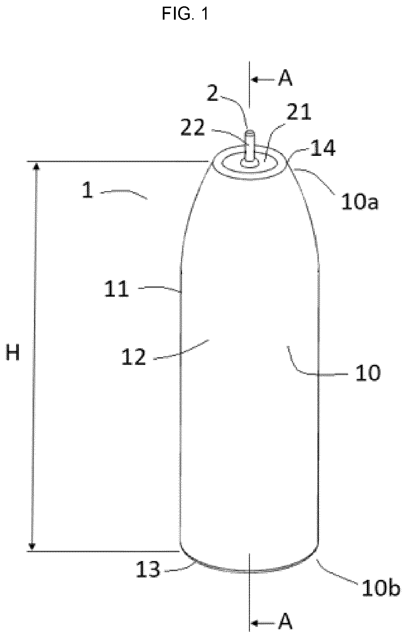

Fig. 1 représente un mode de réalisation d'une cartouche de stockage 1 de gaz utilisée pour conserver, i.e. stocker, conditionner, contenir ou analogue, un mélange gazeux NO/N₂ selon la présente invention. La cartouche de gaz 1 comprend un corps principal 11 formant une enveloppe périphérique définissant un compartiment ou volume interne 12 servant à contenir et stocker le mélange gazeux NO/N₂.

Le corps principal 11 est allongé selon l'axe AA de la cartouche 1. Il présente un fond 13, par exemple une surface plane ou bombée, une portion tubulaire intermédiaire 10 sensiblement en forme d'ogive et une extrémité supérieure 10a fermée par un couvercle 21 portant une valve de distribution 2 ou de dispensation du gaz servant à contrôler la sortie du mélange gazeux du volume interne 12 du corps principal 11. La valve de distribution 2 est dotée d'un canal d'échappement 22 pour acheminer le gaz. La structure et le fonctionnement de la valve de distribution 2 sont détaillés ci-après.

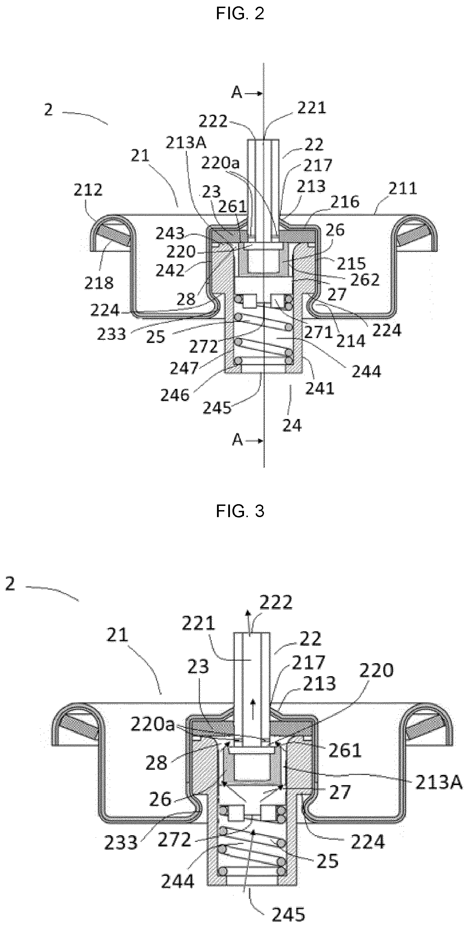

Le couvercle 21 est préférentiellement hermétiquement serti ou soudé au pourtour 14 de la portion tubulaire intermédiaire 10, au niveau de son extrémité supérieure 10a. En cas de sertissage du couvercle 21 sur le pourtour supérieur 14 du corps principal 11, une étanchéité parfaite est réalisée entre eux grâce à un joint torique plat 218, i.e. un joint plat circulaire, inséré entre ces éléments, comme visible en Fig. 2. Par ailleurs, le fond 13 est préférentiellement hermétiquement soudé à l'extrémité inférieure 10b du corps principal 11.

Le corps principal 11 formant l'enveloppe périphérique de la cartouche de gaz 1 est métallique, préférentiellement un alliage d'aluminium, et a une paroi ayant une épaisseur de quelques dixièmes de millimètres, par exemple comprise entre 0,1 et 0,5 mm environ. Il en va de même du couvercle 21 et du fond 13.

La cartouche de gaz 1 peut contenir dans son volume interne 12 un mélange NO/N₂ sous pression faible, c'est-à-dire n'excédant pas une ou quelques dizaines de bar, typiquement moins de 15 bar. Le volume interne 12 de la cartouche de gaz 1 est de dimensions limitées, c'est-à-dire qu'il est inférieur à 1000 mL, de préférence moins de 900 mL, voire moins de 800 mL, par exemple un volume de l'ordre 790 mL.

De préférence, le corps principal 11 de la cartouche de gaz 1 a une hauteur H comprise entre 10 cm et 30 cm, mesurée entre les deux extrémités supérieure 10a et inférieure 10b, i.e. au niveau du fond 13.

Du fait de sa conception simple et ses dimensions compactes, la cartouche de gaz 1 peut être fabriquée à grande échelle, ce qui présente un avantage en termes de facilité de production et donc de coût.

Fig. 2 et Fig. 3 schématisent l'architecture et le fonctionnement d'un mode de réalisation de la valve de distribution 2 de gaz surmontant la cartouche de gaz 1.

La valve de distribution ou dispensation 2 est portée par le couvercle de forme circulaire 21 qui est formé d'une paroi ou enveloppe 211 conformée, c'est-à-dire incluant des portions ou zones spécifiques successives, notamment un pourtour extérieur arrondi 212, un bourrelet central 213 traversé par le canal d'échappement 22, et un bossage latéral 214 agencé à la base du bourrelet central 213.

Le bourrelet central 213 forme une protubérance ou renflement de périphérie cylindrique et faisant saillie axialement (axe AA), sur la surface externe du couvercle 21 en étant dirigé vers l'extérieur, i.e. vers le haut sur Fig. 1, c'est-à-dire se projetant en éloignement au-dessus du couvercle 21 selon l'axe AA. Le bourrelet central 213 définit un compartiment interne 213A. Le bourrelet central 213 est formé par déformation et/ou mise en forme de la pièce formant le couvercle 21, tel un disque métallique.

Le couvercle 21 métallique, de préférence en alliage d'aluminium, présente une épaisseur de quelques dixièmes de millimètres, par exemple de 0,1 à 0,5 mm. Le bourrelet central 213 situé au centre du couvercle 21 présente un évidement traversant ou passage central 217, situé en son centre, logeant le canal d'échappement 22 qui se présente sous forme d'un conduit creux 221. Le canal d'échappement 22 et le couvercle 21 sont coaxiaux, et par ailleurs agencés également coaxialement (axe AA) sur le corps 11 de la cartouche de gaz 1.

Le canal d'échappement 22 comprend une extrémité aval libre 222 situé à l'extérieur et une extrémité amont 220 située dans le compartiment interne 213A du bourrelet central 213. L'extrémité aval libre 222 comprend un orifice de sortie du gaz, alors que l'extrémité amont 220 comprend des canaux d'entrée de gaz latéraux 220a par lesquels le gaz provenant du volume interne 12 de la cartouche 1 peut pénétrer dans le conduit creux 221 pour y circuler ensuite en direction de l'orifice de sortie du gaz, comme visible en Fig. 3.

Le canal d'échappement 22 comprend, à son extrémité amont 220 se prolongeant ou située dans le compartiment interne 213A du bourrelet central 213 du couvercle 21, un élément de siège 26. Autrement dit, l'élément de siège 26 se trouve dans le compartiment interne 213A du bourrelet central 213 du couvercle 21. Selon un mode de réalisation, l'élément de siège 26 se présente ici sous forme d'un corps cylindrique 261 comprenant un évidement central 263 incluant un fond borgne.

La solidarisation du canal d'échappement 22 à l'élément de siège 26 se fait par exemple via un montage en force sur un épaulement 262, par soudage ou tout autre technique. Selon un autre mode de réalisation, le canal d'échappement 22 et l'élément de siège 26 peuvent être formés d'une pièce unique.

Le canal d'échappement 22 est apte à translater dans le passage central du bourrelet central 213 du couvercle 21 en direction du compartiment interne 213A du bourrelet central 213. Le canal d'échappement 22 et l'élément de siège 26 forment ainsi un ensemble ou système de contrôle de la sortie/libération de gaz qui est mobile axialement (axe AA). En effet, la face avant 261 de l'élément de siège 26 assure, lorsqu'elle est en contact avec un élément d'étanchéité, tel un joint torique plat 23, agencé dans le fond 216 du bourrelet central 213, une étanchéité fluidique entre l'élément de siège 26 et l'élément d'étanchéité 23, pour empêcher alors toute sortie de gaz de la cartouche 1, comme illustré en Fig. 2 et détaillé ci-après. A l'inverse, lorsque la face avant 261 de l'élément de siège 26 n'est plus en contact de l'élément d'étanchéité 23, il se crée un espacement entre eux, c'est-à-dire une rupture d'étanchéité, qui permet au gaz de passer et d'entrer dans le conduit creux 221 et y circuler ensuite en direction de l'orifice de sortie du gaz.

Par ailleurs, une pièce-support 24 tubulaire, préférentiellement de forme cylindrique, forme un manchon 241 présentant une paroi extérieure 242 elle-même cylindrique. La pièce-support 24 est insérée dans le compartiment interne 213A du bourrelet central 213 formée dans l'enveloppe 211 du couvercle 21, c'est-à-dire dans la partie interne du bourrelet central 213 du couvercle 21.

Plus précisément, une partie de la paroi extérieure 242 du manchon 241 vient en contact avec une portion latérale interne 215 du bourrelet central 213 du couvercle 21. La pièce-support 24 est alors maintenue en position par un bossage latéral 214 obtenu par déformation radial orienté vers l'axe AA, c'est-à-dire vers l'intérieur du compartiment interne 213A du bourrelet central 213, d'une partie de l'enveloppe 211 ou paroi située à la base du bourrelet central 213 du couvercle 21.

Autrement dit, le bourrelet central 213 comprend, à sa base 233, un bossage latéral 214 permettant de retenir la pièce-support 24 en position fixe dans le compartiment interne 213A du bourrelet central 213, en venant coopérer avec un épaulement 224 situé sur la paroi externe de la pièce-support 24 tubulaire. Le bossage latéral 214 correspond à un enfoncement ou une déformation de la paroi périphérique du bourrelet central 213 (à sa base 233) qui est est dirigé vers l'intérieur du compartiment interne 213A du bourrelet central 213, c'est-à-dire que la paroi interne du compartiment interne 213A du bourrelet central 213 forme, au niveau de sa base 233, une expansion annulaire se projetant dans le compartiment interne 213A et venant coopérer avec l'épaulement 224 situé sur la paroi externe de la pièce-support 24 tubulaire.

La pièce-support 24 tubulaire forme aussi un manchon autour de l'élément de siège 26 situé à l'extrémité interne du canal d'échappement 22 de sorte que, lors des translations du canal d'échappement 22 dans le passage central du bourrelet central 213 du couvercle 21, ledit élément de siège 26 qui est solidaire du canal d'échappement 22, puisse lui aussi translater en même temps au sein du manchon que forme la pièce-support 24 tubulaire, c'est-à-dire dans le logement ou volume interne 244 situé au centre de la pièce-support 24 tubulaire, comme visible sur Fig. 3.

Par ailleurs, la pièce-support 24 comprend une extrémité annulaire 243 venant appuyer sur un élément d'étanchéité, tel un joint torique 23 (i.e. annulaire) plat agencé dans le fond interne 216 du bourrelet central 213 et traversé coaxialement par le canal d'échappement 22. Autrement dit, l'extrémité annulaire 243 de la pièce-support 24 est aussi coaxiale au canal d'échappement 22 et au joint torique 23, i.e. l'élément d'étanchéité, de sorte que le joint torique 23 soit pris en sandwich et comprimé entre l'extrémité annulaire 243 de la pièce-support 24 et le fond interne 216 du bourrelet central 213 du couvercle 21.

L'élément d'étanchéité, tel un joint torique plat 23, est donc maintenu en position par la pièce-support 24 formant manchon, alors que le canal d'échappement 22 peut coulisser en son centre qui est évidé, i.e. joint en forme d'anneau.

Par ailleurs, l'élément de siège 26 est prolongé par un élément diffuseur 27 pouvant lui aussi coulisser dans le volume interne 244 de la pièce-support 24. Cet élément diffuseur 27 est de forme sensiblement cylindrique et présente un cou 271 traversé en son centre par un canal central 272. Le canal central 272 forme alors une liaison fluidique entre le volume interne 244 de la pièce-support 24 et une chambre interne 28 située entre, d'une part, la surface extérieure 262 de l'élément de siège 26 et la surface interne 247 de la pièce-support 24 et, d'autre part, entre le joint torique 23 et l'élément diffuseur 27.

Comme visible sur Fig. 2 et Fig. 3, l'élément diffuseur 27 se trouve aussi dans le compartiment interne 213A du bourrelet central 213 du couvercle 21.

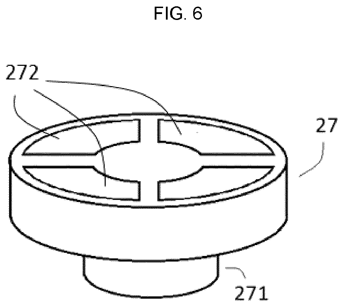

Selon un mode de réalisation, l'élément diffuseur 27 peut se présenter sous la forme d'une structure tubulaire avec un cou 271 et des ouvertures 272 pour le gaz, comme illustré en Figure 6. Le cou 271 de l'élément diffuseur 27 qui est orienté vers le volume interne 12 de la cartouche 1, c'est-à-dire qui se projette axialement (AA) vers l'intérieur de la cartouche 1, permet de recevoir et maintenir en position, l'une des extrémités d'un moyen élastique 25, à savoir ici un ressort de forme cylindrique, qui est logé et comprimé entre l'élément diffuseur 27 et le fond 246 de la pièce-support 24.

Le ressort 25 repousse normalement l'élément diffuseur 27 en direction de l'élément de siège 26 de manière à assurer une étanchéité fluidique entre ledit l'élément de siège 26 et le joint torique 23, comme illustré en Fig. 2.

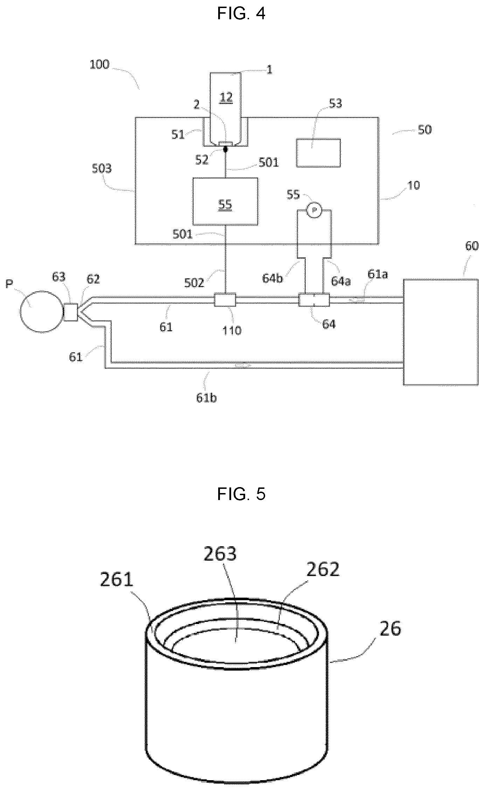

Le fond 246 de la pièce-support 24 est par ailleurs traversé par un canal ou passage axial 245. Il existe donc une communication fluidique entre le passage axial 245 et le volume interne 244 de la pièce-support 24, le canal central 272 de l'élément diffuseur 27 et la chambre interne 28 permettant au flux gazeux, i.e. mélange gazeux NO/N₂, de circuler au travers de ces éléments avant de sortir du volume interne 12 de la cartouche 1 lorsque le gaz est utilisé, i.e. est envoyé à un circuit ventilatoire 61 de ventilateur 60 via un dispositif 50 de délivrance NO, comme schématisé en Fig. 4.

Plus précisément, dans la configuration dite « fermée » ou « de repos » illustrée en Fig. 2, le ressort 25 repousse l'élément diffuseur 27 et par conséquent aussi l'élément de siège 26 de façon à ce que la face avant 261 dudit élément de siège 26 vienne comprimer le joint torique 23 plat et assure ainsi une étanchéité fluidique parfaite entre la chambre interne 28 de la pièce-support 24 et le conduit interne creux 221 du canal d'échappement 22.

Dans cette configuration dite « fermée », aucun gaz n'est délivré par le conduit interne creux 221 du canal d'échappement 22. Autrement dit, le gaz contenu dans le volume interne 12 de la cartouche de gaz 1 est en communication fluidique avec le canal ou passage axial 245 et le volume interne 244 de la pièce-support 24, et se diffuse jusqu'à la chambre interne 28 mais ne peut s'échapper dans le conduit interne creux 221 du canal d'échappement 22 via les canaux d'entrée de gaz latéraux 220a.

A l'inverse, Fig. 3 illustre la valve 2 en configuration dite « ouverte » dans laquelle du gaz est délivré par le conduit interne creux 221 du canal d'échappement 22.

Afin de faire passer la valve 2 de la configuration dite « fermée » de Fig. 2 à la configuration dite « ouverte » de Fig. 3, il suffit d'appliquer sur l'extrémité libre 222 du canal d'échappement 22, une force mécanique forçant ledit canal d'échappement à opérer une translation axiale selon l'axe AA en direction de la cartouche 1 de manière à ce que le canal d'échappement 22 coulisse dans les passages centraux du bourrelet central 213 du couvercle 21 et du joint torique plat 23.

Autrement dit, en appliquant une force extérieure sur l'extrémité libre 222 du canal d'échappement 22 visant à repousser le canal d'échappement 22 tubulaire vers la valve 2 et la cartouche 1, on obtient une libération du gaz qui peut alors passer dans le lumen ou conduit interne creux 221 du canal d'échappement 22 et en ressortir ensuite par l'orifice de sortie qui se trouve au niveau de l'extrémité libre 222 du canal d'échappement 22.

La force extérieure appliquée sur l'extrémité 222 du canal d'échappement 22 peut résulter de l'insertion de la cartouche 1 dans un logement 51 spécifique aménagé dans le dispositif 50 de délivrance NO, lequel logement 51 spécifique comprend un mécanisme d'actionnement 52 (non détaillé) venant coopérer avec la valve de distribution 2 de gaz de cartouche de gaz 1 selon l'invention, comme schématisé en Fig. 4 pour libérer le gaz.

Du fait du couplage existant entre le canal d'échappement 22 et l'élément de siège 26, l'élément de siège 26 subit également un mouvement de translation axiale sur l'axe AA, et repousse alors simultanément l'élément diffuseur 27 en engendrant une compression plus prononcée du ressort à spirales cylindrique 25, comme visible sur Fig. 3.

Ce faisant, la face avant 261 de l'élément de siège 26 perd alors le contact avec le joint torique plat 23 (qui reste en position, comme en Fig.2), ce qui engendre une rupture d'étanchéité fluidique entre eux.

La chambre interne 28 se retrouve alors en communication fluidique non seulement avec le conduit intérieur creux 221 via les canaux d'entrée de gaz latéraux 220a du canal d'échappement 22 mais aussi avec le canal central 272 de l'élément diffuseur 27, le volume interne 244 et le canal 245 de la pièce-support 24. Il peut alors s'établir une circulation de gaz au travers de ces différents éléments et en direction de l'orifice de sortie de gaz porté par l'extrémité libre 222 du canal d'échappement 22, comme illustré sur Fig. 3.

Sur Fig. 3, les flèches schématisent la circulation du gaz depuis le volume interne 12 de la cartouche 1 et au travers de la valve 2 de distribution.

Le gaz contenu dans le volume interne 12 de la cartouche de gaz 1, c'est-à-dire le mélange NO/N₂, se diffuse alors via la chambre interne 28 jusqu'au conduit interne creux 221 du canal d'échappement 22 par lequel il peut ensuite s'échapper et être recueilli par le circuit interne 501 du dispositif 50 de délivrance NO, comme expliqué ci-après et illustré en Fig. 4.

Autrement dit, la cartouche de gaz 1 munie d'une telle valve de distribution 2 est selon l'invention préférentiellement destinée à être insérée dans un logement dédié 51 d'un dispositif de délivrance de NO 50 et à y être maintenue en position en configuration de vanne 2 dite « ouverte » via une contrainte mécanique sur l'extrémité libre 222 du canal d'échappement 22 permettant de le repousser en translation et ainsi libérer le gaz, i.e. le mélange NO/N₂ contenu dans la cartouche 1.

D'une manière générale, à titre d'exemple, on conçoit une cartouche de gaz ayant un volume intérieur 12 de l'ordre 790 ml renfermant un mélange gazeux binaire N₂/NO pressurisé à environ 10 bar mesuré à 23°C environ, ledit mélange N₂/NO contenant environ 23000 ppmv de NO, le reste étant de l'azote, et éventuellement des impuretés inévitables en quantité négligeable, telle de la vapeur d'eau ou de l'oxygène gazeux.

Ceci équivaut à 7900 ml de mélange gazeux N₂/NO à pression atmosphérique (i.e. 1 bar) et correspond en outre à un volume de NO ainsi conditionné d'environ 180 ml pour la teneur considérée de 23000 ppmv de NO.

A titre comparatif, une bouteille de gaz classiquement utilisée pour conditionner les mélange N₂/NO permet de fournir 1963 L d'un mélange N₂/NO ayant une concentration en NO de 800 ppmv. Ceci correspond à un volume disponible de NO 1570 ml, soit un rapport du volume de NO disponible d'environ 10.

La cartouche de gaz 1 selon l'invention offre une simplicité d'utilisation accrue, une logistique simple, un encombrement moindre, engendre des risques d'utilisation (blessure en cas de chute ou de manipulation ou manipulation de produits dangereux) quasi-nuls pour les opérateurs, c'est-à-dire le personnel soignant, et peut être fabriquée plus simplement, donc à un coût très inférieur.

De plus, une cartouche de gaz 1 selon l'invention est par ailleurs conforme aux exigences et réglementations sur le transport et les expéditions des gaz sous pression, ce qui permet de faire transiter ce type de cartouche 1 par un service de messagerie ou de livraison grand public, tel que Fedex ou UPS. Ceci présente aussi un avantage indéniable en termes de logistique et de facilité d'exploitation notamment.

Fig. 4 schématise un mode de réalisation d'une installation 100 de fourniture, i.e. délivrance, d'un gaz contenant du NO à un patient incorporant une cartouche de stockage 1 de gaz utilisée pour stocker un mélange gazeux NO/N₂ selon l'invention, par exemple la cartouche de stockage 1 de Fig. 1.

Cette installation 100 de fourniture de gaz à un patient P comprenant la cartouche de gaz 1 faisant office de source d'un mélange gazeux NO/N₂ contenant entre 15000 et 25000 pppmv de NO (le reste étant de l'azote) selon l'invention, agencée de manière à alimenter, via sa valve de distribution 2 de gaz, la portion amont du circuit de gaz interne 501 d'un dispositif de délivrance 50 de NO conçu pour fournir le mélange gazeux NO/N₂ au circuit ventilatoire 61 d'un ventilateur médical 60.

La cartouche de gaz 1 utilisée selon l'invention vient préférentiellement se loger dans un logement dédié 51 du dispositif de délivrance de NO 50 comprenant un mécanisme d'actionnement 52 (non détaillé) venant coopérer avec la valve de distribution 2 de gaz de cartouche de gaz 1 selon l'invention, notamment en appuyant sur l'extrémité 222 du canal d'échappement 22 de manière à repousser le canal d'échappement 22 tubulaire en direction de la cartouche 1 et à autoriser ainsi le passage du gaz du volume interne 12 de la cartouche de gaz 1 selon l'invention audit circuit de gaz interne 501 du dispositif de délivrance 50 de NO.

Selon un autre mode de réalisation, deux (ou plus) cartouches de gaz 1 identiques peuvent être insérées dans le logement dédié 51 du dispositif de délivrance de NO 50 pour permettre de basculer sur une cartouche 1 pleine lorsque l'autre cartouche 1 qui est en cours d'utilisation, tend à se vider, en assurant ainsi la continuité de la thérapie, c'est-à-dire éviter toute interruption de fourniture de NO au patient P. Autrement dit, dans ce cas, les deux cartouches de gaz 1 sont agencées en parallèle l'une de l'autre de manière à être utilisée de manière alternative. La cartouche vide peut alors être remplacée, pendant que l'autre délivré du gaz, i.e. le mélange NO/N₂. Dans ce cas, le logement dédié 51 du dispositif de délivrance de NO 50 est dimensionné pour accueillir plusieurs cartouches 1 et comprend par ailleurs un mécanisme d'actionnement 52 dédié à chaque cartouche, c'est-à-dire des mécanismes d'actionnement 52 venant coopérer avec la valve de distribution 2 de gaz équipant chaque cartouche de gaz 1.

Dans tous les cas, le raccordement fluidique de la (ou des) cartouche de gaz 1 au dispositif de délivrance de NO 50 se fait de manière étanche grâce à l'utilisation de moyens d'étanchéité, comme des joints toriques ou analogues.

Dans le dispositif de délivrance de NO 50, on prévoit des moyens de contrôle 55 du débit et/ou de la pression du gaz, agencés sur le circuit de gaz interne 501, permettant de contrôler ou ajuster le débit et/ou la pression du gaz convoyé par le circuit de gaz interne 501 du dispositif de délivrance 50 de NO, par exemple un régulateur de pression, une ou des vannes de contrôles, telles des électrovannes, un ou des orifices calibrés, un ou des clapets anti-retour....

Les moyens de contrôle 55 du débit et/ou de la pression du gaz sont pilotés par des moyens de pilotage 53, aussi appelée unité de pilotage, telle une carte électronique à microprocesseur(s) mettant en œuvre un ou des algorithmes ou tout autre système de contrôle adapté.

Le flux gazeux de NO/N₂ en sortie des moyens de contrôle 55 du débit et/ou de la pression du gaz est acheminé par la portion aval du circuit de gaz interne 501 puis un conduit d'injection 502, avant d'être délivré dans la branche inspiratoire 61A du circuit ventilatoire 61 patient connecté fluidiquement à un ventilateur médical 60, à savoir un appareil d'assistance respiratoire fournissant un gaz respiratoire contenant au moins 21% d'oxygène, typiquement de l'air ou un mélange azote/oxygène.

Pour faciliter la compréhension, on considère que le gaz respiratoire fourni par le ventilateur médical 60 est de l'air. Cet air circule dans la branche inspiratoire 61A depuis le ventilateur 60 jusqu'à une interface respiratoire 63, tel un masque ou un sonde trachéale, alimentant le patient P avec le mélange thérapeutique contenant le NO à la teneur souhaitée.

Le flux gazeux de NO/N₂ amené par le conduit d'injection 502 se mélange à l'air directement dans la branche inspiratoire 61A du circuit ventilatoire 61 de manière à obtenir un mélange thérapeutique final contenant essentiellement de l'oxygène, de l'azote et la proportion désirée de NO.

La proportion désirée de NO dépend de la posologie fixée par le médecin, du type de patient (adulte, enfant, nourrisson...), de la pathologie considérée (PPHN, hypertension pulmonaire...), ou autre. En général, le teneur en NO est comprise entre 1 et 80 ppmv de NO dans le mélange thérapeutique final qui est fourni au patient P, via l'interface respiratoire 63, c'est-à-dire un mélange gazeux final contenant essentiellement de l'oxygène, de l'azote et du NO.

Le circuit ventilatoire 61 patient comprend en outre une branche expiratoire 61B pour récupérer les gaz riches en CO₂ expirés par le patient et les convoyer jusqu'au ventilateur médical 1, notamment à des fins d'analyse, avant qu'ils ne soient rejetés à l'atmosphère.

Les branches inspiratoire 61A et expiratoire 61B, tels des tuyaux flexibles, viennent se raccorder à une pièce de liaison 62, appelé pièce en Y, agencé en amont de l'interface respiratoire 63.

Optionnellement, un humidificateur de gaz (non montré) peut être agencé sur la branche inspiratoire 61A pour humidifier le gaz fourni au patient P.

Par ailleurs, il est prévu un capteur de débit 64 agencé sur la branche inspiratoire 61A, entre le ventilateur 60 et le site d'injection de NO fourni par le conduit d'injection 502. Ce capteur de débit 64 peut comprendre une ligne amont 64A et une ligne aval 64B de mesure de pression qui sont connectées fluidiquement au capteur de débit 64 en des sites de raccordement situés en amont et en aval d'une restriction interne, afin d'y opérer les mesures de pression du flux circulant, avant et après perte de charge engendrée par la restriction interne (non détaillé).

Les lignes 64A, 64B forment des conduits de mesure de pression qui fournissent les mesures de pression du flux circulant, avant et après perte de charge, à un capteur de pression P différentiel 55 agencé dans le dispositif de délivrance 50 de NO. Ce capteur de pression différentiel 55 est intégré dans le boitier 503 du dispositif de délivrance de NO 50 et est soit connecté électriquement à l'unité de pilotage 53, soit lui transmet les mesures de pression afin qu'elles y soient traitées informatiquement.

L'unité de pilotage 53 constitue en outre un système de traitement de données, notamment des mesures opérées par les capteurs ou autre, permettant notamment de déterminer le débit du flux d'air circulant dans la branche inspiratoire 61A.

Connaitre ce débit permet aux moyens de pilotage 53 de déterminer la quantité de NO/N₂ à injecter dans le flux d'air pour obtenir la teneur en NO désirée dans le mélange thérapeutique final, c'est-à-dire résultant de l'injection du mélange NO/N₂ dans l'air circulant dans le lumen de la branche inspiratoire 61A. Les moyens de pilotage 53 vont alors coopérer avec les moyens de contrôle 55 du débit et/ou de la pression du dispositif de délivrance 50 de NO pour réguler le flux de mélange NO/N₂ (i.e. débit et/ou pression) alimentant le conduit d'injection 502 fournissant le mélange NO/N₂.

Avoir recours à une cartouche de gaz 1 selon l'invention au sein d'une telle installation 100 de fourniture, i.e. délivrance, d'un gaz contenant du NO à un patient permet de réduire l'encombrement général de l'installation tout en facilitant ses manipulations, notamment les remplacements de la cartouche vide par une cartouche pleine 1, et ce, à coût contrôlé.

Une telle installation peut être utilisée pour fournir des mélanges gazeux à base de NO à des patients, en particulier des adultes, enfants, adolescents ou nouveau-nés, souffrant d'hypertensions pulmonaires et/ou d'hypoxie, qui peuvent engendrer des vasoconstrictions pulmonaires ou analogues, par exemple causés par des pathologies ou troubles pulmonaires de type PPHN (hypertension pulmonaire persistante du nouveau-né) ou SDRA (syndrome de détresse respiratoire aigüe), ou encore engendrés par une opération de chirurgie cardiaque avec mise du patient sous circulation sanguine extracorporelle.

## Revendications

1. Utilisation d'une cartouche de stockage (1) de gaz sous pression comprenant :
- un corps principal (11) comprenant un volume interne (12) compris entre 500 mL et 800 mL pour contenir le mélange gazeux, et
- une valve de distribution (2) pour contrôler la sortie du mélange gazeux du volume interne (12) du corps principal (11),
pour stocker un mélange gazeux NO/N₂ ayant une concentration en NO comprise entre 20 000 et 24 000 ppmv, à une pression inférieure à 11,5 bar, mesurée à 23°C,
ladite cartouche de stockage (1) pesant moins de 1 kg.

2. Utilisation selon la revendication 1, **caractérisée en ce que** la cartouche de stockage (1) pèse entre 150 et 750 grammes.

3. Utilisation selon la revendication 1, **caractérisée en ce que** la concentration en NO dans le mélange gazeux NO/N₂ est comprise entre 22 500 et 23 500 ppmv.

4. Utilisation selon la revendication 1, **caractérisée en ce que** le corps principal (11) a une forme d'ogive.

5. Utilisation selon la revendication 1, **caractérisée en ce que** le corps principal (11) est en alliage d'aluminium.

6. Utilisation selon la revendication 1, **caractérisée en ce que** le corps principal (11) de la cartouche (1) est fermé à son extrémité supérieure (10a) par un couvercle (21), la valve de distribution (2) étant portée par le couvercle (21), de préférence un couvercle (21) en alliage d'aluminium.

7. Utilisation selon la revendication 6, **caractérisée en ce que** :
- le couvercle (21) de la cartouche (1) est conformé pour présenter un bourrelet central (213) comprenant un passage central (217) et définissant un compartiment interne (213A) et
- la valve de distribution (2) de la cartouche (1) comprend un canal d'échappement (22) associé à un élément de siège (26), ledit canal d'échappement (22) étant mobile en translation axiale au sein du passage central (217) du bourrelet central (213), et l'élément de siège (26) coopérant avec un élément d'étanchéité (23) pour opérer une étanchéité gazeuse et empêcher tout passage de gaz du volume interne (12) de la cartouche (1) au canal d'échappement (22).

8. Utilisation selon la revendication 7, **caractérisée en ce que** l'élément de siège (26) est normalement repoussé en direction de l'élément d'étanchéité (23) par un moyen élastique (25), de préférence par l'intermédiaire d'un élément diffuseur (27) agencé entre l'élément de siège (26) et l'élément d'étanchéité (23).

9. Utilisation selon l'une des revendications 7 ou 8, **caractérisée en ce que** l'élément de siège (26) est mobile en translation dans une pièce-support (24) formant un manchon (241) logeant l'élément de siège (26) et le moyen élastique (25), et préférentiellement l'élément diffuseur (27), ladite pièce-support (24) étant logée et fixée dans le compartiment interne (213A) du bourrelet central (213).

10. Utilisation selon la revendication 7, **caractérisée en ce que** le canal d'échappement (22) comprend une extrémité aval libre (222) située à l'extérieur du bourrelet central (213) et une extrémité amont (220) située dans le compartiment interne (213A) du bourrelet central (213) et coopérant avec l'élément de siège (26).

11. Utilisation selon la revendication 1, **caractérisée en ce que** le corps principal a une hauteur comprise entre 15 cm et 30 cm.

12. Utilisation selon la revendication 1, **caractérisée en ce que** le corps principal comprend une paroi ayant une épaisseur comprise entre 0,1 et 0,5 mm.

13. Utilisation selon l'une des revendications précédentes, d'une cartouche de gaz (1) en tant que source de NO pour alimenter en mélange gazeux NO/N₂, un dispositif de délivrance de NO (50) d'une installation (100) de fourniture de gaz à un patient (P) comprenant :
- ladite au moins une source de NO (1),
- ledit dispositif de délivrance de NO (50),
- une branche inspiratoire (61A) d'un circuit patient (61) alimentée en mélange gazeux NO/N₂ par le dispositif de délivrance de NO (50), et
- un ventilateur médical (60) en communication fluidique avec la branche inspiratoire (61A) pour alimenter ladite branche inspiratoire (61A) en un gaz respiratoire contenant au moins 21% d'oxygène.

14. Utilisation selon la revendication 13, **caractérisée en ce que** le gaz respiratoire contenant au moins 21% d'oxygène est de l'air ou un mélange oxygène/azote.

## Patentansprüche

1. Verwendung einer Speicherkartusche (1) für Gas unter Druck, umfassend:
- einen Hauptkörper (11), umfassend ein Innenvolumen (12) zwischen 500 ml und 800 ml, um das Gasgemisch zu enthalten, und
- ein Abgabeventil (2), um das Austreten des Gasgemisches aus dem Innenvolumen (12) des Hauptkörpers (11) zu steuern,
zum Speichern eines NO/N₂-Gasgemisches mit einer NO-Konzentration zwischen 20 000 und 24 000 ppmv bei einem Druck unter 11,5 bar, gemessen bei 23 °C,
wobei die Speicherkartusche (1) weniger als 1 kg wiegt.

2. Verwendung nach Anspruch 1, **dadurch gekennzeichnet, dass** die Speicherkartusche (1) zwischen 150 und 750 Gramm wiegt.

3. Verwendung nach Anspruch 1, **dadurch gekennzeichnet, dass** die NO-Konzentration in dem NO/N₂-Gasgemisch zwischen 22 500 und 23 500 ppmv beträgt.

4. Verwendung nach Anspruch 1, **dadurch gekennzeichnet, dass** der Hauptkörper (11) eine Spitzbogenform hat.

5. Verwendung nach Anspruch 1, **dadurch gekennzeichnet, dass** der Hauptkörper (11) aus Aluminiumlegierung ist.

6. Verwendung nach Anspruch 1, **dadurch gekennzeichnet, dass** der Hauptkörper (11) der Kartusche (1) an seinem oberen Ende (10a) durch eine Abdeckung (21) verschlossen wird, wobei das Abgabeventil (2) von der Abdeckung (21) getragen wird, bevorzugt einer Abdeckung (21) aus Aluminiumlegierung.

7. Verwendung nach Anspruch 6, **dadurch gekennzeichnet, dass**:
- die Abdeckung (21) der Kartusche (1) so ausgebildet ist, dass sie eine zentrale Wulst (213) aufweist, die eine zentrale Durchführung (217) umfasst und einen Innenraum (213A) definiert, und
- das Abgabeventil (2) der Kartusche (1) einen Auslasskanal (22) umfasst, der einem Sitzelement (26) zugeordnet ist, wobei der Auslasskanal (22) in der zentralen Durchführung (217) der zentralen Wulst (213) axial translatorisch beweglich ist und wobei das Sitzelement (26) mit einem Dichtungselement (23) zusammenwirkt, um eine Gasdichtigkeit herzustellen und jeden Durchgang von Gas vom Innenvolumen (12) der Kartusche (1) zum Auslasskanal (22) zu verhindern.

8. Verwendung nach Anspruch 7, **dadurch gekennzeichnet, dass** das Sitzelement (26) normalerweise durch ein elastisches Mittel (25), bevorzugt über ein Diffusorelement (27) das zwischen dem Sitzelement (26) und dem Dichtungselement (23) angeordnet ist, in Richtung des Dichtungselements (23) gedrückt wird.

9. Verwendung nach einem der Ansprüche 7 oder 8, **dadurch gekennzeichnet, dass** das Sitzelement (26) in einem Stützteil (24), das eine Hülse (241) bildet, die das Sitzelement (26) und das elastische Mittel (25) und bevorzugt das Diffusorelement (27) aufnimmt, translatorisch beweglich ist, wobei das Stützteil (24) in dem Innenraum (213A) der zentralen Wulst (213) aufgenommen und befestigt ist.

10. Verwendung nach Anspruch 7, **dadurch gekennzeichnet, dass** der Auslasskanal (22) ein freies stromabwärtiges Ende (222), das außerhalb der zentralen Wulst (213) gelegen ist, und ein stromaufwärtiges Ende (220), das in dem Innenraum (213A) der zentralen Wulst (213) gelegen ist, und mit dem Sitzelement (26) zusammenwirkt, umfasst.

11. Verwendung nach Anspruch 1, **dadurch gekennzeichnet, dass** der Hauptkörper eine Höhe zwischen 15 cm und 30 cm hat.

12. Verwendung nach Anspruch 1, **dadurch gekennzeichnet, dass** der Hauptkörper eine Wand umfasst, die eine Dicke zwischen 0,1 und 0,5 mm hat.

13. Verwendung nach einem der vorhergehenden Ansprüche einer Gaskartusche (1) als NO-Quelle zur Versorgung einer NO-Abgabevorrichtung (50) einer Anlage (100) zur Bereitstellung von Gas für einen Patienten (P) mit NO/N₂-Gasgemisch, umfassend:
- die mindestens eine NO-Quelle (1),
- die NO-Abgabevorrichtung (50),
- einen Einatemzweig (61A) eines Patientenkreislaufs (61), der durch die NO-Abgabevorrichtung (50) mit NO/N₂-Gasgemisch versorgt wird, und
- ein medizinisches Beatmungsgerät (60) in Fluidverbindung mit dem Einatemzweig (61A), um den Einatemzweig (61A) mit einem Atemgas zu versorgen, das mindestens 21 % Sauerstoff enthält.

14. Verwendung nach Anspruch 13, **dadurch gekennzeichnet, dass** das Atemgas, das mindestens 21 % Sauerstoff enthält, Luft oder ein Sauerstoff/StickstoffGemisch ist.

## Claims

1. Use of a cartridge (1) for storing pressurized gas, comprising:
- a main body (11) comprising an internal volume (12) of between 500 ml and 800 ml for containing the gaseous mixture, and
- a distribution valve (2) for controlling the output of the gaseous mixture from the internal volume (12) of the main body (11),
for storing a gaseous mixture NO/N₂ having a concentration of NO of between 20000 and 24000 ppmv, at a pressure of less than 11.5 bar, measured at 23°C,
said storage cartridge (1) weighing less than 1 kg.

2. Use according to Claim 1, **characterized in that** the storage cartridge (1) weighs between 150 and 750 grams.

3. Use according to Claim 1, **characterized in that** the concentration of NO in the gaseous mixture NO/N₂ is between 22500 and 23500 ppmv.

4. Use according to Claim 1, **characterized in that** the main body (11) has an ogive shape.

5. Use according to Claim 1, **characterized in that** the main body (11) is made of aluminium alloy.

6. Use according to Claim 1, **characterized in that** the main body (11) of the cartridge (1) is closed at its upper end (10a) by a lid (21), the distribution valve (2) being carried by the lid (21), preferably a lid (21) made of aluminium alloy.

7. Use according to Claim 6, **characterized in that**:
- the lid (21) of the cartridge (1) is configured to have a central bead (213) comprising a central passage (217) and defining an internal compartment (213A), and
- the distribution valve (2) of the cartridge (1) comprises an escape channel (22) associated with a seat element (26), said escape channel (22) being movable in axial translation within the central passage (217) of the central bead (213), and the seat element (26) cooperating with a sealing element (23) in order to obtain gas leaktightness and prevent any passage of gas from the internal volume (12) of the cartridge (1) to the escape channel (22).

8. Use according to Claim 7, **characterized in that** the seat element (26) is normally pushed back in the direction of the sealing element (23) by an elastic means (25), preferably by way of a diffuser element (27) arranged between the seat element (26) and the sealing element (23).

9. Use according to either of Claims 7 and 8, **characterized in that** the seat element (26) is movable in translation in a support component (24) forming a sleeve (241) housing the seat element (26) and the elastic means (25), and preferably the diffuser element (27), said support component (24) being housed and fixed in the internal compartment (213A) of the central bead (213).

10. Use according to Claim 7, **characterized in that** the escape channel (22) comprises a free downstream end (222) situated outside the central bead (213), and an upstream end (220) situated in the internal compartment (213A) of the central bead (213) and cooperating with the seat element (26).

11. Use according to Claim 1, **characterized in that** the main body has a height of between 15 cm and 30 cm.

12. Use according to Claim 1, **characterized in that** the main body comprises a wall having a thickness of between 0.1 and 0.5 mm.

13. Use, according to one of the preceding claims, of a gas cartridge (1) as NO source for feeding a gaseous mixture NO/N₂ to an NO supply device (50) of an installation (100) for delivering gas to a patient (P), comprising:
- said at least one NO source (1),
- said NO supply device (50),
- an inhalation branch (61A) of a patient circuit (61) fed with a gaseous mixture NO/N₂ by the NO supply device (50), and
- a medical ventilator (60) in fluidic communication with the inhalation branch (61A) in order to feed said inhalation branch (61A) with a respiratory gas containing at least 21% of oxygen.

14. Use according to Claim 13, **characterized in that** the respiratory gas containing at least 21% of oxygen is air or an oxygen/nitrogen mixture.
